# EUROPEAN PATENT SPECIFICATION

(11) **EP 0 396 353 B1**
(45) Date of publication and mention of the grant of the patent: **15.03.1995**
(21) Application number: 90304624.1
(22) Date of filing: 27.04.1990
(51) Int. Cl.: A61M 25/10, A61B 5/03

(54) **Inflation/deflation device for angioplasty catheter including a housing mounted digital display**
Aufblas-/Entleerungsvorrichtung für Angioplastie-Katheter mit einer am Gehäuse angeordneten digitalen Anzeige
Dispositif de gonflage/dégonflage pour un cathéter d'angioplastie comportant un dispositif d'affichage monté sur le boîtier

(30) Priority: 02.05.1989 US 346221
(43) Date of publication of application: 07.11.1990
(73) Proprietor: SCHNEIDER (USA) INC., Plymouth, Minnesota 55442 (US)
(72) Inventor: Shockey, Rick L., Eagan, Minnesota (US)
(74) Representative: Hayles, James Richard

(56) References cited:
- EP-A- 0 290 770
- US-A- 4 370 982
- US-A- 4 723 938
- US-A- 4 815 313

## Description

This invention relates generally to apparatus for performing angioplasty procedures for opening partially occluded blood vessels, and more particularly to a hand-operated inflation and fluid dispensing device adapted to be connected to the proximal end of an angioplasty catheter for either inflating the expander member on that catheter or supplying a radiopaque contrast medium through the catheter and out its distal end whereon a digital display of inflation pressure is mounted to the housing of the syringe.

There is discussed in Schjeldahl et al. U.S. Patent Nos. 4,723,938 and 4,758,223 inflation/deflation syringes for use with an angioplasty catheter. The teachings of U.S. Patent Nos. 4,723,938 and 4,758,223 are incorporated herein by this reference in their entirety. Such syringes may include mechanical pressure measuring gauges mounted to the syringe outlets. Because such gauges are mechanical in nature, they are cumbersome to work with and add weight to the syringe as compared with lighter weight and smaller electronic devices. Further, such mechanical measuring gauges are usually not illuminated and are, therefore, hard to read when being used due to the fact that most cardiovascular operating rooms are dimly lit when the syringes are in use.

There is currently on the market an angioplasty catheter inflation device in the form of a molded plastic housing configured to contain a hypodermic-type syringe which includes a disposable pressure transducer for sensing pressure during inflations and deflations. The device is being marketed by Merit Medical Systems, Inc., Salt Lake City, Utah, under the trademark Intelliflator™. The Intelliflator™ device includes a cable which is connected from the transducer and syringe to an external electronic box which displays data, such as a 24-hour clock, the date, duration of the inflation, time elapse since the last inflation and inflation pressure in PSI or atmospheres. Such a device, however, requires complicated and expensive microprocessor-controlled electronics which are contained within the separate display box. Further, the physician using the syringe must contend with the extra cable required to connect the transducer to the display box. Further still, when using such a system, the physician must divert his eyes from the point of application of the syringe whenever he desires to observe the pressure reading on the display box.

US Patent no. 4,815,313 discloses a pressure calibration device for calibrating pressure transducers at relatively low gauge pressures by producing a known reference pressure.

The present invention provides an inflation/deflation syringe apparatus for selectively injecting and withdrawing a fluid from a balloon catheter comprising a balloon inflation/deflation syringe device including a generally tubular housing having an outlet, a pressure sensing means mounted within the housing in fluid communication with the outlet, a display means for displaying the sensed pressure that is connected to the pressure sensing means and mounted to the housing and locking means for locking the syringe at a selected pressure, characterised in that, the pressure sensing means is a pressure transducer that is electrically connected to the display means, the display means provides a digital readout of the sensed pressure and is mounted to the housing by a display pedestal by which the display is angled back from the distal end of the housing, a converting means for converting the pressure scale from PSI to atmospheres is mounted to the housing and is electrically connected to the display means, and the locking means is mounted to the housing.

In accordance with the present invention, the foregoing shortcomings of the prior art inflators have been eliminated by conveniently incorporating in a single housing a pressure transducer, a digital display for the pressure and a housing mounted switch for converting the scale displayed to atmospheres. The inflation/deflation syringe apparatus of the invention includes a balloon catheter inflation/deflation syringe device including a generally tubular housing having an outlet. A pressure transducer is mounted within the housing in fluid communication with the outlet and means for displaying a digital readout of the sensed pressure is electrically connected to the pressure transducer and also mounted to the housing. Since the display and the conversion switch are both mounted to the housing, it is not necessary for the physician using the device to divert his eyes from the syringe while it is in use in order to observe a pressure reading. In some embodiments of the device, the display electronics may be backlighted or may comprise an LED display to allow the user an easily discernible and readable display. In some applications, an illuminated display may not be necessary.

It is accordingly a principal object of the present invention to provide an improved inflation/deflation device for a transluminal angioplasty catheter with a housing mounted digital display.

Another object of the invention is to provide an inflation/deflation device for a transluminal angioplasty catheter which includes a digital display without the need for external cables or display panels.

Yet another object of the invention is to provide a convenient hand-held device which can be used to introduce pressures within the expander member of a transluminal angioplasty catheter without requiring the user to divert his sight away from the point of application of the syringe in order to observe pressure readings.

These and other objects and advantages of the invention will become apparent to those skilled in the art from the following detailed description of a preferred embodiment, especially when considered in conjunction with the accompanying drawings in which like numerals in several views refer to corresponding parts.

Figure 1 is a top elevation view of a preferred embodiment of the invention.

Figure 2 is a side view of the embodiment of Figure 1.

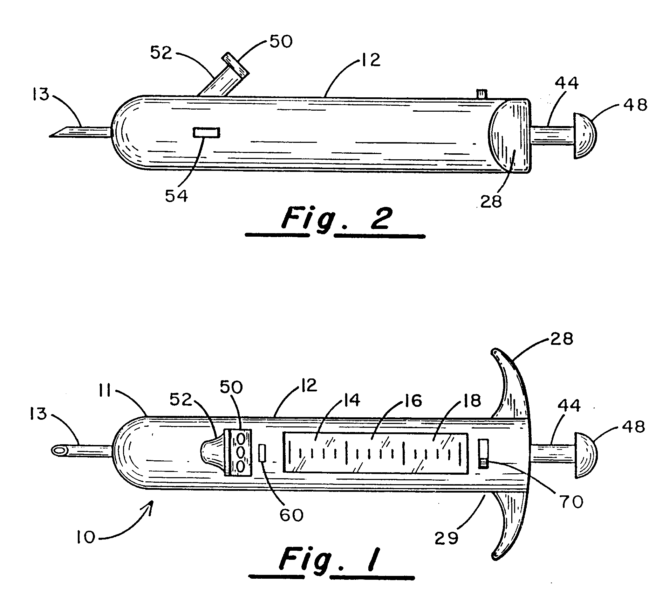

Referring to Figure 1, the inflation/deflation syringe apparatus for a balloon catheter is indicated generally by numeral 10 and is seen to include a generally tubular housing 12 which is preferably formed in a molding process from a suitable plastic material. An elongated slot or opening 14 is formed through the side wall of the housing, allowing the user to view the graduated markings 16 formed on the exterior surface of a tubular syringe 18 contained within the bore of the housing 12. It is believed that such tubular syringes are quite conventional and include a cylindrical chamber in which is disposed a reciprocally movable piston member (not shown) which is formed of an elastomeric material and dimensioned so as to provide a seal with the side walls of the cylindrical chamber. The syringe further includes an end cap 28 and a knob 48 affixed to the proximal end of an elongated shaft 44.

A digital display means 50 is mounted to the housing 12. As is best shown in Figure 2, the housing 12 includes a display pedestal 52 which is angled back from the distal end 11 of the housing. The pedestal 52 may advantageously be an integral part of the housing, a separate plastic piece part or a part which is integrated into the digital display unit. The inflation/deflation syringe apparatus further includes transducer 54 which is electrically connected to the display 50 through conductors (not shown) which are inserted through a hollow portion of pedestal 52. Transducer 54 is mounted within the housing 12 disposed to be in fluid communication with the outlet 13 of the syringe. Such transducers and associated displays suitable for mounting in the syringe housing are conventionally available and the electrical connections necessary are well known and are usually supplied by the manufacturer of the transducer devices. The display unit may advantageously be an LCD display such as the type used in conventionally available digital wrist watches, LED display, a backlighted LCD-type display or other equivalent display device. Small batteries typically provide power to run such displays and are usually included in the display package, or may be stored in the syringe housing or pedestal.

Also electrically connected to the display is a conversion switch 60. The conversion switch 60, when activated, will switch the display mode pressure scale from pounds-per-square inch (PSI) to atmospheres (atm) depending on the preference of the user. A locking switch 70 is also included at the proximal end 29 of the housing for locking in the plunger at a selected pressure.

In operation, the user would apply the inflation/deflation syringe apparatus to an angioplasty catheter, inflating the catheter by gripping the syringe with both hands having one hand gripping the housing and the other hand with fingers wrapped around the end cap 28 while the palm of the hand is used to press on the knob 48. The user can readily view the pressure reached during this operation by observing the digital display 50 without the need for averting his eyes from the treatment site. When the desired pressure is reached, the physician may lock the syringe at that pressure by using locking switch 70. When it is again desired to deflate the balloon or aspirate the treatment site, the physician reverses the procedure still able to observe the pressure readings without the need for looking away from the treatment site.

This invention has been described herein in considerable detail in order to comply with the Patent Statutes and to provide those skilled in the art with the information needed to apply the novel principles and to construct and use such specialized components as are required. However, it is to be understood that the invention can be carried out by specifically different equipment and devices and that various modifications, both as to equipment details and operating procedures, can be accomplished without departing from the scope of the invention itself as defined in the claims.

## Claims

1. An inflation/deflation syringe apparatus (10) for selectively injecting and withdrawing a fluid from a balloon catheter comprising a balloon inflation/deflation syringe device (18) including a generally tubular housing (12) having an outlet (13), a pressure sensing means (54) mounted within the housing (12) in fluid communication with the outlet (13), a display means (50) for displaying the sensed pressure that is connected to the pressure sensing means (54) and mounted to the housing (12), and locking means (70) for locking the syringe (18) at a selected pressure, characterised in that, the pressure sensing means (54) is a pressure transducer that is electrically connected to the display means (50), the display means provides a digital readout of the sensed pressure and is mounted to the housing (12) by a display pedestal (52) by which the display is angled back from the distal end (11) of the housing (12), a converting means (60) for converting the pressure scale to atmospheres is mounted to the housing (12) and is electrically connected to the display means (50), and the locking means (70) is mounted to the housing (12).

2. An apparatus as claimed in Claim 1 wherein the display means (50) is illuminated.

3. An apparatus as claimed in Claim 1 wherein the display means (50) comprises an LCD digital display.

4. An apparatus as claimed in Claim 1 wherein the display means (50) comprises an LED digital display.

5. An apparatus as claimed in Claim 1 wherein the display means (50) comprises a backlighted LCD display.

## Patentansprüche

1. Aufblas-/Entleerungs-Spritzenapparatur (10) zum selektiven Injizieren und Abziehen eines Fluids aus einem Ballonkatheter, umfassend eine Ballonaufblas-/entleerungs-Spritzenvorrichtung (18) einschließlich eines im allgemeinen rohrförmigen Gehäuses (12) mit einem Auslaß (13), ein Druckfühlelement (54), das innerhalb des Gehäuses (12) in Fluidkommunikation mit dem Auslaß (13) montiert ist, ein Anzeigeelement (50) zum Anzeigen des erfühlten Druckes, das mit dem Druckfühlelement (54) verbunden und an dem Gehäuse (12) montiert ist, und ein Arretierelement (70) zum Arretieren der Spritze (18) bei einem gewählten Druck, dadurch gekennzeichnet, daß das Druckfühlelement (54) ein Druckwandler ist, der elektrisch an das Anzeigeelement (50) angeschlossen ist, das Anzeigeelement eine digitale Ablesung des erfühlten Druckes liefert und an dem Gehäuse (12) durch einen Anzeigesockel (52) montiert ist, durch welchen die Anzeige vom distalen Ende (11) des Gehäuses (12) zurückgewinkelt ist, ein Umwandlungselement (60) zum Umwandeln der Druckskala in Atmosphären an dem Gehäuse (12) montiert und elektrisch an das Anzeigeelement (50) angeschlossen ist und das Arretierelement (70) an dem Gehäuse (12) montiert ist.

2. Apparatur nach Anspruch 1, bei der das Anzeigeelement (50) beleuchtet ist.

3. Apparatur nach Anspruch 1, bei der das Anzeigeelement (50) ein digitales LCD-Display umfaßt.

4. Apparatur nach Anspruch 1, bei der das Anzeigeelement (50) ein digitales LED-Display umfaßt.

5. Apparatur nach Anspruch 1, bei der das Anzeigeelement (50) ein LCD-Display mit Hintergrundbeleuchtung umfaßt.

## Revendications

1. Appareil (10) à seringue de gonflage/dégonflage pour sélectivement injecter un fluide dans un cathéter à ballonnet et retirer un fluide de ce cathéter, comportant un dispositif (18) à seringue de gonflage/dégonflage de ballonnet comprenant un boîtier globalement tubulaire (12) ayant une sortie (13), un moyen capteur de pression (54) monté à l'intérieur du boitier (12) en communication de fluide avec la sortie (13), un moyen d'affichage (50) destiné à afficher la pression captée qui est raccordé au moyen capteur de pression (54) et monté sur le boîtier (12), et un moyen de verrouillage (70) destiné à verrouiller la seringue (18) à une pression choisie, caractérisé en ce que le moyen capteur de pression (54) est un transducteur de pression qui est connecté électriquement au moyen d'affichage (50), le moyen d'affichage (50) fournit une indication numérique de la pression captée et est monté sur le boitier (12) à l'aide d'une colonnette (52) d'affichage par laquelle l'affichage est incliné vers l'arrière de l'extrémité distale (11) du boîtier (12), un moyen (60) de conversion destiné à convertir l'échelle de pression en atmosphère est monté sur le boîtier (12) et est connecté électriquement au moyen d'affichage (50), et le moyen de verrouillage (70) est monté sur le boîtier (12).

2. Appareil selon la revendication 1, dans lequel le moyen d'affichage (50) est éclairé.

3. Appareil selon la revendication 1, dans lequel le moyen d'affichage (50) comporte un affichage numérique à cristaux liquides.

4. Appareil selon la revendication 1, dans lequel le moyen d'affichage (50) comporte un affichage numérique à diodes électroluminescentes.

5. Appareil selon la revendication 1, dans lequel le moyen d'affichage (50) comporte un affichage à cristaux liquides éclairé par l'arrière.
